(19) Europäisches Patentamt
European Patent Office
Office européen des brevets

(11) Veröffentlichungsnummer: **0 196 275 B1**

(12) **EUROPÄISCHE PATENTSCHRIFT**

(45) Veröffentlichungstag der Patentschrift: **23.09.92**

(51) Int. Cl.$^5$: **C07D 251/70**

(21) Anmeldenummer: **86810131.2**

(22) Anmeldetag: **20.03.86**

(54) **Verfahren zur Herstellung von 2,4,6-Tris-(amino-phenylamino)-triazinen.**

(30) Priorität: **26.03.85 CH 1315/85**

(43) Veröffentlichungstag der Anmeldung:
**01.10.86 Patentblatt 86/40**

(45) Bekanntmachung des Hinweises auf die Patenterteilung:
**23.09.92 Patentblatt 92/39**

(84) Benannte Vertragsstaaten:
**BE CH DE FR GB LI**

(56) Entgegenhaltungen:
**EP-A- 0 074 926**
**EP-A- 0 094 642**
**EP-B- 0 038 299**
**DE-A- 2 513 731**

**CHEMICAL ABSTRACTS, Band 58, Nr. 5, 04. März 1963, Columbus, Ohio, US; ITARU HONDA (FUKUI UNIV.): "Arylmelamines. II.Diarylmelamines." Spalte 4568, Zusammenfassung-Nr. 4568c**

(73) Patentinhaber: **CIBA-GEIGY AG**
**Klybeckstrasse 141**
**CH-4002 Basel(CH)**

(72) Erfinder: **Ronco, Roland, Dr.**
**Moosackerweg 12**
**CH-4148 Pfeffingen(CH)**
Erfinder: **Stingelin, Willy**
**Stockackerstrasse 1A**
**CH-4153 Reinach(CH)**
Erfinder: **Hänggi, Ernst**
**Im Höfli 13**
**CH-4125 Riehen(CH)**

EP 0 196 275 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 2,4,6-Tris-(amino-phenylamino)-triazinen, durch Kondensation von s-Trichlortriazin mit drei Aequivalenten partiell geschütztem Phenylendiamin und anschliessendes Abspalten der Schutzgruppen.

2,4,6-Tris-(amino-phenylamino)-triazine sind wichtige Zwischenprodukte für Textil-, Leder- und Papierfarbstoffe sowie für Pigmente. Ueber die Diaminophenylen-Brücken können bis zu drei farbige Reste an den Triazinkern gebunden werden. Man erhält so vor allem wertvolle Substantivfarbstoffe, die sich insbesondere zum Färben von Baumwolle und Papier eignen (DE-A-32 23 436).

2,4,6-Trihalogentriazine zeigen eine ähnliche Reaktivität wie Säurehalogenide (Houben-Weyl, Methoden der organischen Chemie Bd. 5/4 (1960), Seite 712). Um Nebenreaktionen zu vermeiden werden daher nucleophile Substitutionen am halogenierten Triazinkern üblicherweise in wasserfreien aprotischen Lösungsmitteln, wie z.B. Toluol, Halogenbenzolen oder Nitrobenzol durchgeführt (siehe z.B. DE-A-2011043). Nachteil dieses Verfahrens ist jedoch, dass das Reaktionsprodukt in diesen Lösungsmitteln im allgemeinen gut löslich ist, was sich ungünstig auf dessen Aufarbeitung auswirkt. Angestrebt wird eine Verfahrensführung, bei der das geschützte Tris-(aminophenylamino)-triazin am Ende der Reaktion als Feststoff im Reaktionsmedium vorliegt und daraus beispielsweise durch eine einfache Filtration isoliert werden kann.

Aufgabe der vorliegenden Erfindung ist es daher, ein neues Verfahren bereitzustellen, wonach das durch Umsetzung von s-Trichlortriazin mit drei Aequivalenten eines geschützten Phenylendiamins gebildete Produkt in suspendierter Form im Reaktionsmedium vorliegt.

Gefunden wurde, dass aliphatische Alkohole mit 2 bis 4 C-Atomen als Lösungsmittel dieser Anforderung genügen. 2,4,6-Tris-(amino-phenylamino)-triazine sind praktisch unlöslich in derartigen Alkoholen und können am Ende der Reaktion auf einfache Weise nach bekannten Methoden vom Lösungsmittel abgetrennt werden. Anschliessend erfolgt dann die Abspaltung der Schutzgruppen. Eine Reaktion des Alkohols mit dem Trichlortriazin wird praktisch nicht beobachtet. Bereits das disubstituierte Triazin ist nur wenig löslich in den $C_2$- bis $C_4$-Alkoholen und kann daher ausfallen, was sich jedoch überraschenderweise nicht negativ auf die Ausbeute an dem gewünschten 3-fach substituierten Produkt, dem geschützten Tris-(amino-phenylamino)-triazin auswirkt.

Gegenstand der vorliegenden Erfindung ist somit ein Verfahren zur Herstellung von 2,4,6-Tris-(amino-phenylamino)triazinen durch Kondensation von s-Tri-chlortriazin mit drei Aequivalenten eines partiell geschützten, gegebenenfalls durch Halogen, Cyan, $C_1$-$C_4$-Alkyl oder $C_1$-$C_4$-Alkoxy substituierten m-oder p-Phenylendiamins und anschliessendes Abspalten der Schutzgruppen, dadurch gekennzeichnet, dass man die Kondensation in einem wasserfreien oder bis 10% Wasser enthaltenden aliphatischen Alkohol mit 2 bis 4 C-Atomen in Gegenwart eines säurebindenden Mittels durchführt, wobei dieses säurebindende Mittel mindestens in solcher Menge umgesetzt wird, die ausreicht, die frei werdende Salzsäure zu neutralisieren.

Unter 2,4,6-Tris-(amino-Phenylamino)-triazinen werden im vorliegenden Fall Verbindungen der folgenden Struktur verstanden:

(1)

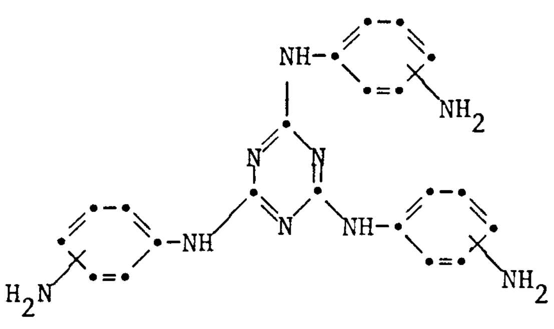

wobei der aromatische Kern der einzelnen Diaminophenylenreste noch ein- oder mehrfach, durch Halogen, wie Fluor, Chlor, Brom oder Jod, durch die Cyano-gruppe, insbesondere jedoch durch Alkyl($C_1$-$C_4$) oder Alkoxy($C_1$-$C_4$), wie Methyl, Aethyl, Propyl, iso-Propyl oder Butyl bzw. Methoxy, Aethoxy, Propoxy, iso-Propoxy oder Butoxy substituiert sein kann. Die Aminogruppe ist jeweils in m- oder vor allem in p-Stellung zum Aminobrückenglied (-NH-) angeordnet.

Umgesetzt wird das s-Trichlortriazin mit einem partiell geschützten Phenylendiamin, d.h. einem gegebenenfalls substituierten m- oder p-Phenylendiamin, dessen eine Aminogruppe durch eine Schutzgruppe blockiert ist. Während der Kondensationsreaktion wird durch ein säurebindendes Mittel die freiwerdende

Salzsäure neutralisiert, so dass das Reaktionsgemisch einen pH-Wert im Bereich von 7 hat, d.h. als Schutzgruppen kommen sowohl sauer, wie auch basisch abspaltbare Schutzgruppen in Betracht, verwiesen wird auf J.F.W. McOmie, Protective groups in organic chemistry, Plenum Press 1973. In erster Linie werden im erfindungsgemässen Verfahren monoacylierte Phenylendiamine eingesetzt. In Frage kommen vor allem die Acylreste niederaliphatischer Carbonsäuren oder auch aromatischer Carbonsäuren, wie z.B. der Formyl-, Acetyl- oder Benzoylrest; zur Blockierung einer der Aminogruppen der Phenylendiamine wird vor allem die Acetylgruppe verwendet.

Wie bereits erwähnt, wird das partiell geschützte Phenylendiamin in mindestens der 3-fach molaren Menge bezogen auf s-Trichlortriazin eingesetzt. Um einen möglichst vollständigen Umsatz zu erreichen, erweist es sich als vorteilhaft, das partiell geschützte Phenylendiamin mit einem Ueberschuss von 10 bis 15 Gew.-% bezogen auf die 3-fach molare Menge zu verwenden.

Vorzugsweise wird als partiell geschütztes Phenylendiamin 4-Aminoacetanilid eingesetzt.

Als aliphatische Alkohole mit 2 bis 4 C-Atomen kommen neben Aethanol, Propanol, und Butanol auch die entsprechenden sekundären und tertiären Alkohole, wie z.B. Isopropanol, Isobutanol und tert.-Butanol in Frage. Als Reaktionsmedium bevorzugt ist Aethanol. Gute Ausbeuten werden auch mit Isopropanol und n-Butanol als Lösungsmittel erhalten.

Durch Anwesenheit von Wasser im Alkohol lässt sich die Ausbeute günstig beeinflussen. Zweckmässigerweise verwendet man Alkohole mit einem Wassergehalt von 1 bis 10 Gew.-%, insbesondere 3 bis 7 Gew.-%, das entspricht etwa handelsüblichen technischen Alkoholen. Natürlich kann man auch von reinen $C_2$ bis $C_4$ Alkoholen oder Alkoholen mit geringerem Wassergehalt ausgehen und durch Zugabe von Wasser den zur Erzielung einer optimalen Ausbeute erforderlichen Wassergehalt einstellen. Bei Verwendung von Alkoholen mit einem Wassergehalt über 10 Gew.-% lässt sich das 2-fach substituierte Triazin, das geschützte Di-(amino-phenylamino)-chlortriazin, nur noch bedingt zum gewünschten Tris-(amino-phenylamino)-triazin umsetzen. Die Umsetzung in absolutem Alkohol ist möglich, liefert allgemein jedoch eine um ca. 10 % schlechtere Ausbeute, als bei Verwendung von Alkohol mit einem geringen Wassergehalt.

Als säurebindendes Mittel werden im vorliegenden Verfahren beispielsweise Alkali- oder Erdalkalimetallcarbonate, Metalloxide, auch Alkalimetallsalze organischer Säuren, wie z.B. Alkaliacetate oder auch Alkalialkoholate, wie z.B. Natriumethylat, verwendet; in Frage kommen ferner aromatische Amine, wie z.B. Pyridin. Bevorzugt wird als säurebindendes Mittel Natriumethylat oder wasserfreies Natriumcarbonat, insbesondere mit einer Korngrösse von mehr als 100 Mikrometer verwendet.

Eingesetzt wird das säurebindende Mittel in einer Menge, die ausreicht, die pro Mol s-Trichlortriazin frei werdenden 3 Mol Salzsäure zu neutralisieren, wobei zweckmässigerweise mit einem Ueberschuss von 10 bis 20 Gew.-% gearbeitet wird.

Was die Reaktionstemperatur anbetrifft, so kann man diese nach Zusammengeben von partiell geschütztem Phenylendiamin und s-Trichlortriazin durch die freiwerdende Reaktionswärme zunächst auf ca. 40° bis 60°C ansteigen lassen und erhitzt dann das Reaktionsgemisch, je nach Siedepunkt des Alkohols vorteilhaft auf eine Temperatur von 60° bis 130°C. Zweckmässigerweise erhitzt man das Reaktionsgemisch unter Rückfluss, bis vollständiger Umsatz erreicht ist. Beim Arbeiten mit Aethanol als Lösungsmittel führt man die Reaktion vorteilhaft bei einer Temperatur von 40° bis 80°C durch. Die Reaktionszeit beträgt allgemein 10 bis 20 Stunden. Der Fortgang der Reaktion lässt sich z.B. durch dünnschichtchromatographische Analyse leicht verfolgen.

Bei der Durchführung des Verfahrens geht man zweckmässigerweise so vor, dass man das partiell geschützte Phenylendiamin zusammen mit dem säurebindenden Mittel im Alkohol vorlegt und die gesamte Menge s-Trichlortriazin auf ein Mal zugibt. Vor Zugabe des Trichlortriazins werden das geschützte Diamin und das säurebindende Mittel vorteilhaft zu einer homogenen Suspension verrührt. Um die Bildung von Hydrolyse- bzw. Oxidationsprodukten möglichst weitgehend zu vermeiden, kann man die Reaktion unter Inertgas durchführen, z.B. in einer Stickstoffatmosphäre.Natürlich kann die Zugabe der einzelnen Komponenten auch in umgekehrter Reihenfolge erfolgen. So kann man beispielsweise das s-Trichlortriazin zusammen mit dem säurebindenden Mittel in Alkohol vorlegen und anschliessend das partiell geschützte Phenylendiamin zugeben.

Das an den Aminogruppen geschützte Tris-(amino-phenylamino)-triazin wird am Ende der Reaktion mittels bekannter Verfahren, beispielsweise durch Filtrieren, zentrifugieren oder Dekantieren aus dem Reaktionsgemisch isoliert und mit Alkohol und Wasser gewaschen. Das noch feuchte Produkt - eine Trocknung ist nicht erforderlich - wird anschliessend zur Abspaltung der Schutzgruppen üblicherweise einer sauren Hydrolyse unterworfen. Dazu erwärmt man das Produkt z.B. in verdünnter Schwefelsäure während ca. 10 Stunden auf eine Temperatur von ca. 90° bis 110°C. Die Schwefelsäure hat zweckmässigerweise eine Konzentration von 5 bis 40 Gew.-%. Nach vollständiger Abspaltung der Schutzgruppen wird das 2,4,6-Tris-(amino-phenylamino)-triazin abfiltriert und zur vollständigen Entfernung der Schwefelsäure z.B. mit

wässrigem Alkalihydroxid behandelt. Schliesslich filtriert man das Produkt ab wäscht salzfrei und trocknet.

Die folgenden Beispiele dienen der Veranschaulichung der Erfindung; Teile bedeuten Gewichtsteile und Prozente Gewichtsprozente. Die NMR-spektroskopische Analyse des Produkts erfolgt unter Verwendung von $d_6$-DMSO als Lösungsmittel; die δ-Werte sind angegeben in ppm relativ zu TMS als innerem Standard.

Beispiel 1: In einem beheizbaren Reaktor mit Rückflusskühler werden in 510 Teilen 94 %igen Aethanol 100 Teile 4-Aminoacetanilid und 37 Teile wasserfreies grobkörniges Natriumcarbonat (Korngrösse 100-200 Mikrometer) vorgelegt und zu einer homogenen Suspension verrührt. In die Suspension, die eine Temperatur von 20° C aufweist, gibt man unter Rühren auf ein Mal 39 Teile s-Trichlortriazin. Die Kondensationsreaktion setzt sofort ein und die Temperatur des Reaktionsgemisches steigt durch die freiwerdende Reaktionswärme auf 40° bis 55° C an. Dann erwärmt man das Reaktionsgemisch weiter auf eine Innentemperatur von 60 bis 70° C und rührt die Reaktionsmasse während 14 Stunden bei dieser Temperatur. Anschliessend lässt man den Reaktorinhalt auf Raumtemperatur abkühlen und filtriert das Kondensationsprodukt ab. Der Filterkuchen wird mit Aethanol und Wasser gewaschen. Man erhält 211 Teile feuchtes Rohprodukt, das 105 Teile 2,4,6-Tris-(4'-acetylamino-phenylamino)-triazin enthält. Das NMR-Spektrum zeigt Signale mit den folgenden chemischen Verschiebungen: $\delta$ = 2,08 (Singulett) Acetyl-$CH_3$; $\delta$ = 7,65 (pseudo Quartett) Phenylenprotonen; $\delta$ = 9,12 (Singulett) und $\delta$ = 9,85 (Singulett) NH-Protonen - Intensitätsverhältnis 3:4:1:1.

Zur Abspaltung der Acetylgruppen wird das feuchte Produkt (211 Teile) in eine Mischung aus 940 Teilen Eis und 400 Teilen 98 %ige Schwefelsäure eingetragen. Anschliessend erhitzt man das Gemisch auf 100° C. Nach 12 Stunden sind die Acetylgruppen vollständig abgespalten und man lässt das Reaktionsgemisch auf Raumtemperatur abkühlen und filtriert das Produkt ab. Das Rohprodukt wird mit Wasser gewaschen und zur Entfernung von Nebenprodukten und Säureresten in 1900 Teilen Wasser und 140 Teilen 30 %iger Natronlauge suspendiert und unter Rückfluss gekocht. Dann filtriert man das Produkt bei 80° C ab und wäscht den Filterkuchen mit heissem Wasser. Nach dem Trocknen erhält man 70 Teile 2,4,6-Tris-(4'-amino-phenylamino)-triazin. Die Ausbeute beträgt 81 %, bezogen auf s-Trichlortriazin.

Beispiel 2: In 510 Teilen 94 %-igem Aethanol werden 100 Teile 4-Aminoacetanilid und 47 Teile Natriumethylat homogen gerührt und bei 20° C mit 39 Teilen s-Trichlortriazin vermengt. Die Kondensation setzt sofort ein und führt zu einem Temperaturanstieg auf 40-45° C. Für vollständigen Umsatz erwärmt man für 16 Stunden auf Rückflusstemperatur. Das auf 20° C gekühlte Reaktionsgemisch wird filtriert und mit Aethanol und Wasser gewaschen.

Die Abspaltung der Schutzgruppe erfolgt analog Beispiel 1.

Das Endprodukt setzt sich zusammen aus:

1 % disubstituiertem und
82 % trisubstituiertem Triazin, bezogen auf eingesetztes Trichlortriazin.

Beispiel 3: In 510 Teilen Isopropanol werden 100 Teile 4-Aminoacetanilid und 37 Teile Natriumcarbonat homogen gerührt und bei 20° C mit 39 Teilen s-Trichlortriazin vermengt. Die Kondensation setzt sofort ein und führt zu einem Temperaturanstieg auf 40-45° C. Für vollständigen Umsatz erwärmt man für 20 Stunden auf Rückflusstemperatur. Das auf 20° C gekühlte Reaktionsgemisch wird filtriert und mit Isopropanol und Wasser gewaschen.

Die Abspaltung der Schutzgruppe erfolgt analog Beipsiel 1.

Das Endprodukt setzt sich zusammen aus:

0 % disubstituiertem und
85 % trisubstituiertem Triazin, bezogen auf eingesetztes Trichlortriazin.

Beispiel 4: In 510 Teilen n-Butanol werden 100 Teile 4-Aminoacetanilid und 37 Teile Natriumcarbonat homogen gerührt und bei 20° C mit 39 Teilen s-Trichlortriazin vermengt. Die Kondensation setzt sofort ein und führt zu einem Temperaturanstieg auf 40-45° C. Für vollständigen Umsatz erwärmt man für 18 Stunden auf 75-80° C und 5 Stunden auf 110° C. Das auf 20° C gekühlte Reaktionsgemisch wird filtriert und mit n-Butanol und Wasser gewaschen.

Die Abspaltung der Schutzgruppe erfolgt analog Beispiel 1.

Das Endprodukt setzt sich zusammen aus:

0 % disubstituiertem und
66 % trisubstituiertem Triazin, bezogen auf eingesetztes Trichlortriazin.

Beispiel 5: In 510 Teilen absolutem Aethanol werden 100 Teile 4-Aminoacetanilid und 58 Teile Natriumcarbonat homogen gerührt und bei 20° C mit 39 Teilen s-Trichlortriazin vermengt. Die Kondensation setzt sofort ein und führt zu einem Temperaturanstieg auf 40-45° C. Für vollständigen Umsatz erwärmt man für 18 Stunden auf Rückflusstemperatur. Das auf 20° C gekühlte Reaktionsgemisch wird filtriert und mit Aethanol und Wasser gewaschen.

Die Abspaltung der Schutzgruppe erfolgt analog Beispiel 1.

Das Endprodukt setzt sich zusammen aus:

0 % disubstituiertem und

72 % trisubstituiertem Triazin, bezogen auf eingesetztes Trichlortriazin.

Beispiel 6: In 510 Teilen 91 %igem Aethanol werden 100 Teile 4-Aminoacetanilid und 58 heile Natriumcarbonat homogen gerührt und bei 20 ° C mit 39 Teilen s-Trichlortriazin vermengt. Die Kondensation setzt sofort ein und führt zu einem Temperaturanstieg auf 40-45 ° C. Für vollständigen Umsatz erwärmt man für 18 Stunden auf Rückflusstemperatur. Das auf 20 ° C gekühlte Reaktionsgemisch wird filtriert und mit Aethanol und Wasser gewaschen.

Die Abspaltung der Schutzgruppe erfolgt analog Beispiel 1.

Das Endprodukt setzt sich zusammen aus:

0 % disubstituiertem und

68 % trisubstituiertem Triazin, bezogen auf eingesetztes Trichlortriazin.

## Patentansprüche

**1.** Verfahren zur Herstellung von 2,4,6-Tris-(amino-phenylamino)-triazinen durch Kondensation von s-Trichlortriazin mit drei Aequivalenten eines partiell geschützten, gegebenenfalls durch Halogen, Cyan, $C_1$-$C_4$-Alkyl oder $C_1$-$C_4$-Alkoxy substituierten m- oder p-Phenylendiamins und anschliessendes Abspalten der Schutzgruppen, dadurch gekennzeichnet, dass man die Kondensation in einem wasserfreien oder bis 10% Wasser enthaltenden aliphatischen Alkohol mit 2 bis 4 C-Atomen in Gegenwart eines säurebindenden Mittels durchführt, wobei dieses säurebindende Mittel mindestens in solcher Menge umgesetzt wird, die ausreicht, die frei werdende Salzsäure zu neutralisieren.

**2.** Verfahren gemäss Anspruch 1, dadurch gekennzeichnet, dass man Aethanol, Isopropanol oder n-Butanol verwendet.

**3.** Verfahren gemäss Anspruch 2, dadurch gekennzeichnet, dass man als Alkohol Aethanol verwendet.

**4.** Verfahren gemäss den Ansprüchen 1 bis 3, dadurch gekennzeichnet, dass ein Alkohol mit einem Wassergehalt von 1 bis 10 Gew.-% verwendet wird.

**5.** Verfahren gemäss Anspruch 4, dadurch gekennzeichnet, dass ein Alkohol mit einem Wassergehalt von 3 bis 7 Gew.-% verwendet wird.

**6.** Verfahren gemäss den Ansprüchen 1 bis 5, dadurch gekennzeichnet, dass man als säurebindendes Mittel ein Alkali- oder Erdalkalimetallcarbonat, das Alkalimetallsalze einer organischen Säure, ein Alkalialkoholat oder Pyridin verwendet.

**7.** Verfahren gemäss Anspruch 6, dadurch gekennzeichnet, dass man als säurebindendes Mittel Natriumethylat oder wasserfreies Natriumcarbonat, insbesondere solches mit einer Korngrösse von mehr als 100 Mikrometer, verwendet.

**8.** Verfahren gemäss Anspruch 1, dadurch gekennzeichnet, dass man die Reaktion bei einer Temperatur von 40 ° C bis 130 ° C durchführt.

**9.** Verfahren gemäss einem der Ansprüche 1 bis 8, dadurch gekennzeichnet, dass man als partiell geschütztes Phenylendiamin 4-Aminoacetanilid verwendet.

**10.** Die nach dem Verfahren gemäss Anspruch 1 hergestellten 2,4,6-Tris-(amino-phenylamino)-triazine.

**11.** Verwendung der gemäss Anspruch 1 erhaltenen 2,4,6-Tris-(aminophenylamino)-triazine zur Herstellung von Textil-, Leder- oder Papierfarbstoffen oder Pigmenten.

## Claims

**1.** A process for the preparation of 2,4,6-tris(aminophenylamino)triazines by condensing s-trichlorotriazine with three equivalents of a partially protected m- or p-phenylenediamine which is optionally substituted by halogen, cyano, $C_1$-$C_4$alkyl or $C_1$-$C_4$alkoxy and subsequently removing the protective groups, which process comprises carrying out the condensation in an aliphatic alcohol which has 2 to 4 C atoms and

is anhydrous or contains up to 10% of water in the presence of an acidbinding agent which is employed in an amount sufficient to neutralise the hydrochloric acid formed.

2. A process according to claim 1, wherein ethanol, isopropanol or n-butanol is used.

3. A process according to claim 2, wherein the alcohol used is ethanol.

4. A process according to any one of claims 1 to 3, wherein the alcohol used contains 1 to 10% by weight of water.

5. A process according to claim 4, wherein the alcohol used contains 3 to 7% by weight of water.

6. A process according to any one of claims 1 to 5, wherein the acid-binding agent used is an alkali metal carbonate or an alkaline earth metal carbonate, an alkali metal salt of an organic acid, an alkali metal alcoholate or pyridine.

7. A process according to claim 6, wherein the acid-binding agent used is sodium ethylate or anhydrous sodium carbonate, which preferably has a particle size of more than 100 micrometres.

8. A process according to claim 1, wherein the reaction is carried out at a temperature from 40°C to 130°C.

9. A process according to any one of claims 1 to 8, wherein the partially protected phenylenediamine used is 4-aminoacetanilide.

10. A 2,4,6-tris(aminophenylamino)triazine obtained by a process as claimed in claim 1.

11. Use of the 2,4,6-tris(aminophenylamino)triazines obtained according to claim 1 for the preparation of textile, leather or paper dyes or pigments.

**Revendications**

1. Procédé pour la préparation de 2,4,6-tris(aminophénylamino)-triazines par condensation de la trichlorotriazine-s avec 3 équivalents d'une m- ou p-phénylènediamine éventuellement substituée par un atome d'halogène ou par un groupe cyano, alkyle en $C_1$-$C_4$ ou alcoxy en $C_1$-$c_4$, partiellement protégée, et élimination subséquente des groupes protecteurs, caractérisé en ce que l'on effectue la condensation dans un alcool aliphatique ayant de 2 à 4 atomes de carbone, anhydre ou contenant jusqu'à 10 % d'eau, en présence d'un agent capteur d'acide, cet agent capteur d'acide étant mis en réaction au moins en une quantité suffisante pour neutraliser l'acide chlorhydrique libéré.

2. Procédé selon la revendication 1, caractérisé en ce que l'on utilise l'éthanol, l'isopropanol ou le n-butanol.

3. Procédé selon la revendication 2, caractérisé en ce que l'on utilise comme alcool l'éthanol.

4. Procédé selon les revendications 1 à 3, caractérisé en ce que l'on utilise un alcool ayant une teneur en eau de 1 à 10 % en poids.

5. Procédé selon la revendication 4, caractérisé en ce que l'on utilise un alcool ayant une teneur en eau de 3 à 7 % en poids.

6. Procédé selon les revendications 1 à 5, caractérisé en ce que l'on utilise, comme agent capteur d'acide, un carbonate de métal alcalin ou alcalino-terreux, un sel alcalin d'un acide organique, un alcoolate alcalin ou la pyridine.

7. Procédé selon la revendication 6, caractérisé en ce que l'on utilise, en tant qu'agent capteur d'acide, de l'éthylate de sodium ou du carbonate de sodium anhydre, en particulier celui ayant une taille de particules de plus de 100 µm.

**8.** Procédé selon la revendication 1, caractérisé en ce que l'on effectue la réaction à une température de 40 à 130 ° C.

**9.** Procédé selon l'une des revendications 1 à 8, caractérisé en ce que, en tant que phénylènediamine partiellement protégée, on utilise le 4-aminoacétanilide.

**10.** Les 2,4,6-tris(aminophénylamino)-triazines préparées par le procédé selon la revendication 1.

**11.** Utilisation des 2,4,6-tris(aminophénylamino)triazines obtenues selon la revendication 1, pour la préparation de colorants ou pigments pour textiles, cuir ou papier.